# EUROPÄISCHE PATENTSCHRIFT

(11) **EP 0 615 442 B1**
(45) Veröffentlichungstag und Bekanntmachung des Hinweises auf die Patenterteilung: **17.09.1997**
(21) Anmeldenummer: 92924548.8
(22) Anmeldetag: 04.12.1992
(51) Int. Cl.: A61K 9/20, A61K 9/16

(54) **SOL-GESTEUERTE THERMOKOLLOIDMATRIX AUF GELATINE BASIS FÜR PERORALE RETARDFORMEN**
SOL-CONTROLLED GELATINE-BASED THERMOCOLLOIDAL MATRIX FOR PERORAL PROLONGED ACTION ADMINISTRATION FORMS
MATRICE THERMOCOLLO DALE REGLEE PAR DES SOLS A BASE DE GELATINE UTILE DANS DES FORMES D'ADMINISTRATION PERORALE A ACTION PROLONGEE

(30) Priorität: 05.12.1991 DE 4140192
(43) Veröffentlichungstag der Anmeldung: 21.09.1994
(73) Patentinhaber: ALFATEC-PHARMA GmbH, 69120 Heidelberg (DE)
(72) Erfinder: WUNDERLICH, Jens-Christian, D-6900 Heilderberg (DE); SCHICK, Ursula, D-6908 Wiesloch (DE); WERRY, Jürgen, D-6700 Ludwigshafen (DE); FREIDENREICH, Jürgen, D-6905 Schriesheim (DE)
(74) Vertreter: Fürniss, Peter, Dr.
(86) Internationale Anmeldenummer: DE9201011
(87) Internationale Veröffentlichungsnummer: WO9310764

(56) Entgegenhaltungen:
- EP-A- 0 138 216
- EP-A- 0 230 654
- EP-A- 0 282 020
- WO-A-85/05029
- WO-A-89/03207
- WO-A-91/06292
- GB-A- 1 516 348
- GB-A- 2 176 105
- US-A- 2 183 053
- US-A- 2 756 177
- US-A- 3 312 594
- AMERICAN PHARMACEUTICAL ASSOCIATION / PHARMACEUTICAL SOCIETY OF GREAT BRITAIN, "Handbook of pharmaceutical excipients", 1986, American Pharm. Assoc., Washington, US, p. 119, paragraph 5

## Beschreibung

Die vorliegende Erfindung bezieht sich auf eine perorale Arzneiform mit konstanter Wirkstoff-Freigabe, die die in vivo Arzneimittelsicherheit für pharmazeutische Wirkstoffe verbessert.

Retardierung eines Wirkstoffes in einer pharmazeutischen Darreichungsform (im engeren Sinne Peroralia) ist dann erwünscht, wenn die biologische Halbwertszeit kurz ist (in der Regel 10 - 12 Stunden). Durch lang anhaltende Freigabe aus der Arzneiform erhofft man sich verschiedene Vorteile:
1. Verbesserte Wirkung
   Möglichst genaue Einstellung von Plasmaspiegeln im therapeutischen Niveau soll einerseits Plasmaspiegelschwankungen, andererseits Nebenwirkungen (u.U. toxisch) vermeiden.
2. Verlängerte Wirkung
   Damit verbunden ist analog eine Reduktion der Einnahmehäufigkeit mit entsprechender Erhöhung der Patienten-Compliance.

Bei Retardarzneimitteln ist die biopharmazeutische Qualität - mit den Parametern Bioverfügbarkeit und in-vitro-Freisetzungsverhalten - besonders wichtig, denn sie enthalten gegenüber Einzeldosierungen relativ hohe Wirkstoffdosen. Weist der galenische Steuermechanismus Mängel auf, können Wirksamkeit wie auch Verträglichkeit beeinträchtigt werden. Neben einer konstanten Wirkstoff-Freigabe 0. Ordnung ist ein pH-unabhängiges Freisetzungsverhalten sicherzustellen.

Wie problematisch der bisherige Stand der Technik ist, soll anhand einiger ausgewählter Beispiele dargestellt werden.

Neben der nur in Einzelfällen möglichen Retardierung durch Komplexe, Salze und Adsorbate oder die Veränderung der Teilchengröße bzw. der Kristallmodifikation des Arzneistoffes, kommen heute im wesentlichen zwei Prinzipien für die Steuerung der Wirkstoff-Freigabe zur Anwendung:
1. Das Überziehen von Wirkstoffen/Arzneiformen mit Filmen
   und
2. die Matrixeinbettung.

Beim Filmcoating werden verschiedene Filmbildner (üblicherweise synthetische, unterschiedlich lösliche oder permeable Polymere auf Polyacrylatbasis) verwendet. Flüssigkeit dringt beispielsweise über Poren in diese Membranen ein und bewirkt eine verlangsamte Freisetzung des gelösten Wirkstoffes durch die diffusionshemmende Polmerbarriere.

Bei der Matrixeinbettung ist der Wirkstoff in ein Gerüst (vor allem aus Polymeren und Hilfsstoffgemischen) eingelagert. Während der Magen-Darm-Passage einer entsprechenden Arzneiform wird der Wirkstoff mehr oder weniger kontrolliert freigesetzt oder aus der gesamten Matrix ausgelaugt, wobei das zurückbleibende unverdauliche Gerüst ausgeschieden wird.

Bedingt durch die Komplexität der Einflußgrößen und Prozeßparameter gelingt es mit den genannten Methoden nur unvollkommen, über einen längeren Zeitraum konstante Plasma- bzw. Gewebespiegel aufrechtzuerhalten. Neben verfahrensbedingten Ursachen spielen intra- und interindividuelle Schwankungen, Verweildauer der Arzneiform in den verschiedenen Bereichen des Verdauungstraktes, Nahrungsaufnahme etc. oft eine entscheidende Rolle. Vor allem bei schwerlöslichen Wirkstoffen mit Säure-Base-Eigenschaften kann es bei ungeeignetem Retardierungsprinzip zu Rekristallisation und Ausfällung des Wirkstoffes in der Arzneiform selbst kommen, was eine erhebliche Einschränkung der Bioverfügbarkeit verursachen kann.

Eine zunächst vielversprechende neuere Entwicklung stellt das sogenannte OROS-System (Orales Osmotisches therapeutisches System) dar, bei dem der Wirkstoff aufgrund des osmotischen Drucks im Innern über eine mikrofeine Abgabeöffnung kontrolliert freigesetzt werden soll. Das System kann neben anderen Nachteilen schwerwiegende Magenirritationen bis hin zu Perforationen verursachen. Untersuchungen haben ergeben, daß dieser sogenannte "Schweißbrennereffekt" durch die extrem bioadhäsiven Eigenschaften der Darreichungsform begünstigt wird.

In letzter Zeit sind quellende Polymere für Hydrogel/Hydrokolloid-Matrixtabletten als geeignetes Verfahren zur kontrollierten Beeinflussung der Wirkstoff-Freigabe zunehmend in der Diskussion. Es ist bekannt, daß sich als hydrophile Gelbildner z.B. Alginate, Cellulosederivate unterschiedlicher Viskosität, wie auch Polyacrylsäuren eignen. Die genannten Stoffe bilden nach Quellung eine Gelschicht aus, die zur Retardierung genutzt werden kann.

Auf Basis von Na-Alginat ist für den Wirkstoff Verapamil-HCl eine solche Formulierung vorgestellt worden, die unter in-vitro-Testbedingung eine annähernd konstante Freigabe 0.Ordnung aufweisen soll. Allerdings können für das Präparat nur spezielle Alginate mit bestimmten Viskositätswerten eingesetzt werden, die zudem nur durch aufwendige Herstellung zu gewinnen sind. Zur Ausformung der Tablette werden zusätzlich weitere Hilfsstoffe von fast bist zu einem Drittel des Gesamtgewichtes benötigt.

Obwohl es mit der Hydrokolloid-Matrixtechnik bei richtiger Auswahl des Polymers in Abstimmung auf den Arzneistoff durchaus gelingen kann, eine kontrollierte Wirkstoff-Freigabe in vitro im Einzelfall zu erzielen, weisen diffusionsabhängige Hydrokolloidsysteme nach neueren Untersuchungen eine lineare Freisetzung erst bei höherer Umdrehungszahl im Dissolutiontest auf.

In vivo können u.a. Wechselwirkungen der einzelnen Polymere mit der gastrointestinalen Flüssigkeit bzw. der eingenommenen Nahrung auftreten und zusätzlich durch die Magen-Darm-Peristaltik verursachter unkontrollierbarer mechanischer Abrieb der aufgequollenen Tabletten-Gelschicht erfolgen.

GB-A-2 176 105 beschreibt Formkörper zum Einsatz in der Nase, die einen Peptidarzneistoff und Gerüstbildner, z. B. Gelatine, enthalten. Aus der Gelatine wird ein schwammartiger Körper hergestellt, aus dessen Hohlräumen die Wirksubstanz herausgelöst wird. Die Auflösung des Gelatineschwamms ist nicht geschwindigkeitsbestimmend für die Auflösung des Wirkstoffs.

US-A-3 312 594 beschreibt eine Depotformulierung in Form eines Komprimats aus gleichen Teilen Pektin, Gelatine und Carboxymethylcellulose sowie dem Wirkstoff für orale Zwecke zur Anwendung in der Mundhöhle.

EP-A-0-230 654 bezieht sich auf Polymere allgemeiner Art, die zum Aufbau einer gepulsten, mehrschichtigen Depotformulierung verwendet werden können. Die beschriebenen Zusammensetzungen zeigen keine lineare Arzneistoffreigabe, sondern eine zeitlich gepulste, wobei die Arzneiform noch von einem unlöslichen Überzug umgeben ist.

WO-A-8 903 207 beschreibt Mikrokapseln, z.B. aus Gelatine, die mit Wirkstoffen beladen sind. Die Wirkstoffe werden durch Diffusion aus den Mikrokapseln freigesetzt; die Freisetzungsrate wird über den Vernetzungsgrad dieser Mikrokapseln gesteuert.

EP-A-0 138 216 beschreibt Präparationen mit verzögerter Freigabe, die Gelatine, einen Peptidarzneistoff und Weichmacher enthalten. Sie sind für die parenterale Gabe oder als Implantat oder zum Einsetzen in den Körper vorgesehen.

WO-A-8 505 029 offenbart ebenfalls eine mikroverkapselte Arzneizubereitung. Um eine Retardierung zu erzielen, werden die Mikrokapseln durch Hitze oder Aldehyde vernetzt und damit gehärtet.

WO-A-9 106 292 beschreibt eine sich schnell auflösende Arzneiform, nämlich einen hydrophoben/aerophilen Feststoff, der in Wasser Mikropartikel ausbildet. Diese besitzen somit keine gesteuerte Freisetzungscharakteristik.

GB-A-1 516 348 beschreibt injizierbare Arzneimittel, die aus Nanopartikeln bestehen. Das Matrixmaterial, z.B. Gelatine wird vernetzt, und das Arzneimittel vorzugsweise parenteral verabreicht.

EP-A-0 282 020 beschreibt perorale Arzneimittel, die als Wirksubstanz saure, nicht steroide entzündungshemmende Mittel und als Trägersubstanz ein Proteinhydrolysat oder ein Polypeptid mit einem mittleren Molekulargewicht von 4000 bis 12000 besitzen. Die beschriebenen Trägersubstanzen sind schnell auflösend (kaltwasserlöslich) und können daher keine Depotwirkung erzielen.

US-A-2 183 053 beschreibt die Herstellung von Vitaminpräparaten in Gelatine-Mikrokapseln.

US-A-2 756 177 beschreibt vitaminhaltige Präparate in Form von Mikrotropfen, zu deren Herstellung die einzelnen Tropfen in einer Stärkepulver-Masse fixiert werden, bis die Teilchen eine feste Form angenommen haben. Offensichtlich sind die beschriebenen Vitaminpräparate keine Depotarzneimittel.

Der Erfindung liegt die Aufgabe zugrunde, ein Depotarzneimittel mit konstanter Wirkstofffreigabe zu schaffen, das die im Stand der Technik auftretenden Nachteile vermeidet. Insbesondere soll ein perorales Depotarzneimittel für leicht- und schwerlösliche, lipophile und hydrophile Arzneistoffe geschaffen werden, das eine über Zeit und Verlauf steuerbare Freisetzung des Arzneistoffes ermöglicht.

Diese Aufgabe wird durch ein perorales Depotarzneimittel gelöst, das gekennzeichnet ist durch ein in wässrigem Medium oberhalb 37°C pro Zeiteinheit annähernd konstant Arzneistoff freisetzendes Komprimat, das im wesentlichen aus einer Matrix aus Gelatine oder fraktionierter Gelatine bzw. deren Mischungen und darin verteiltem Arzneistoff besteht. Diese Aufgabe wird witer durch ein Verfahren zur Herstellung eines solchen peroralen Depotarzneimittels gelöst, das dadurch gekennzeichnet ist, daß man mit einer Gelatine und/oder fraktionierter Gelatine, die sich im wässrigen Medium oberhalb 37°C langsam auflöst, eine pulverförmige Gelatine-Arzneistoff-Mischung herstellt und die Mischung komprimiert. Weiterhin wird die Aufgabe erfindungsgemäß gelöst durch die Verwendung eines in wässrigem Medium oberhalb 37 °C pro Zeiteinheit annähernd konstant Arzneistoff freisetzenden Komprimates aus einer Matrix aus Gelatine und/oder fraktionierte Gelatine und darin verteiltem Arzneistoff zur Herstellung einer peroralen Depotarzneiform. Bevorzugte Ausführungsformen der Erfindung werden in den Unteransprüchen genannt.

Weitere internationale (PCT)-Patentanmeldungen der ALFATEC-Pharma GmbH, gegebenenfalls auch der PAZ Arzneimittelentwicklungsgesellschaft mbH, von demselben Tage betreffen die pharmazeutische Applikation von Nanosolen und ihre Herstellung (DE-A-4140195), die Akutform von 2-Arylpropionsäurederivaten (DE-A-4140185), die Retardform von Dihydropyridinderivaten (DE-A-4140194), die Akutform von S- und R-Ibuprofen (DE-A-4140179), die Retardform von S- und R-Ibuprofen (DE-A-4140172), die Akutform von S- und R-Flurbiprofen (DE-A-4140184), die Retardform von S- und R-Flurbiprofen (DE-A-4140183), die Retardform von 3-Indolylessigsäurederivaten (DE-A-4140191) bzw. die perorale Applikation von Peptidarzneistoffen (DE-A-4140186). Ihre Offenbarung wird auch zum Gegenstand der Offenbarung der vorliegenden Patentanmeldung gemacht.

Erfindungsgemäß wird ein perorales Depotarzneimittel auf Gelatinebasis geschaffen, das eine Sol-gesteuerte lineare Arzneistoffliberation aufweist, die pH-unabhängig und ohne unkontrollierte Erosion bzw. Abrieb erfolgt. Der Arzneistoff liegt in der vorgeschlagenen Matrix vor eindringender gastrointestinaler Flüssigkeit geschützt vor und geht ohne vorherige Diffusion aus der Sol-Form des Polymers direkt in Lösung.

Somit kann mit der erfindungsmäßen Arzneimittel-Depotform die kontrollierte Freisetzung zeitlich variiert und sehr präzise mit hoher Chargenkonformität reproduzierbar eingestellt werden.

Die Erfindung erhöht zusätzlich die Arzneimittelsicherheit und Haltbarkeit. Für Arzneistoffe mit problematischer Bioverfügbarkeit bzw. für Arzneistoffe, die noch nicht peroral zugänglich sind, werden neue Lösungen aufgezeigt.

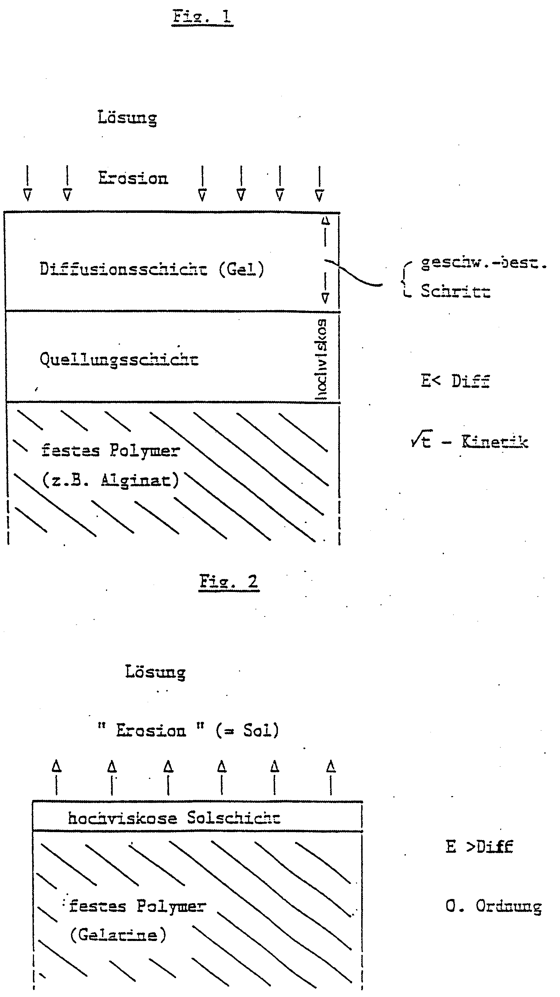

Der erfindungsgemäß gegenüber dem Stand der Technik auftretende Mechanismus der Arzneistoffliberation wird in den Figuren 1 und 2 schematisch verdeutlicht.
- Fig. 1: zeigt schematisch die Freisetzung des in einem festen Polymer, z.B. Alginat, eingebetteten Arzneistoffs unter physiologischen Bedingungen nach dem Stand der Technik. Das feste Polymer, z.B. Alginat, bildet in wäßrigem Medium zwei Schichten aus, die aus einer hochviskosen Quellungsschicht und der Diffusionsschicht (Gel) bestehen. Neben der Erosion erfolgt zeitgleich im Magen-Darm-Trakt die diffusionsabhängige Arzneistoffliberation. Somit entspricht die Freigabe des Arzneistoffs zeitlich einem √t-Gesetz, bei dem die Erosion kleiner ist als die Diffusionsgeschwindigkeit (E < Diff) des Arzneistoffs. Da die Diffusion den geschwindigkeitsbestimmenden Schritt darstellt, ist eine kontrollierte Freigabe sowohl von der Quellungsschicht als auch der Erosion zusätzlich abhängig. Die gelförmige Diffusions- und viskose Quellungsschicht ist zudem mechanisch instabil.
- Fig. 2: zeigt schematisch die Freisetzung des Arzneistoffs gemäß der vorliegenden Erfindung. Das feste Polymer, die Gelatine, in die der Arzneistoff eingebettet ist, bildet unter gleichen Bedingungen eine hochviskose Solschicht aus, die nur geringe Dicke besitzt. Die Arzneistoffliberation erfolgt ausschließlich durch kontinuierliches Auflösen dieser Schicht (E > Diff), die sich fortlaufend mit gleicher Schichtdicke aufbaut. Der Arzneistoff wird bei diesem Vorgang konstant nach nullter Ordnung freigesetzt und ist nicht von zusätzlichen Einflußfaktoren abhängig. Ein vorgelagerter Diffusionsprozeß entfällt und die mechanische Stabilität ist gewährleistet.

Gelatine ist ein bekannter pharmazeutischer Hilfsstoff, der ein ausgeprägtes thermoreversibles Sol-Gelbildungsverhalten in Abhängigkeit von der molekularen Zusammensetzung aufweist. Erst in letzter Zeit ist es durch Einsatz von HPLC und anderen Methoden gelungen, die molekularen Feinstrukturen aufzuklären und die Eigenschaften und das Verhalten der verschiedenen Molekulargewichtsfraktionen zu definieren. Diese Tatsache ermöglicht es heute, für die verschiedenen Anwendungszwecke standardisierte Sorten zum Einsatz zu bringen, die dem natürlichen Biopolymer Gelatine neue technologische Bereiche erschließen.

Mit der vorgestellten Erfindung gelingt es zum ersten Mal, eine retardierte Matrixtablette auf Gelatinebasis reproduzierbar und mit neuen Eigenschaften vorzuschlagen, die zu einer deutlichen Verbesserung des Standes der Technik und der Arzneimittelsicherheit führt. Weiterhin werden spezielle Gelatinesorten erfindungsgemäß eingesetzt, die u.a. besonders peptidarm sind und enge Molekulargewichtsverteilungen zeigen. Es können auch Gelatinederivate und fraktionierte Gelatine geeignet sein.

Spezielle Gelatineherstellungsverfahren und deren Eigenschaften können aus den o.g. Patentanmeldungen vom gleichen Tag entnommen werden.

Eine in der Aufgabe der Erfindung beschriebene Retardform erhält man bereits dadurch, daß man eine feinstkörnig gemahlene handelsübliche Gelatine (Bloomwert 220, Maximum der Molekulargewichtsverteilung bei 100 kD) und einen Arzneistoff auf eine Korngröße von 70 µm klassiert, eine Pulvermischung aus Arzneistoff, Gelatinepulver und üblichen Tablettierhilfsstoffen herstellt und mit einem definierten Restfeuchtegehalt direkt zur Tablette verpreßt.

Erstaunlicherweise hat es sich gezeigt, daß alle Gelatinen, die ein Maximum der Molekulargewichtsverteilung im Bereich von 10⁴ bis 10⁷ D aufweisen, geeignet sind, den Arzneistoff in wäßrigem Medium bei 37°C retardiert über mehrere Stunden linear freizusetzen. Diese Tatsache überrascht umso mehr, da sich bekannterweise Hartgelatinekapseln in wenigen Minuten unter gleichen Testbedingungen vollständig auflösen.

Untersuchungen an den im Stand der Technik erwähnten Hydrokolloid-Matrixtabletten zeigen auf, daß eine Vielzahl von Phänomenen bei der Wirkstoff-Freisetzung polymerhaltiger Arzneiformen auftreten. So wird eine eindeutige Aussage über den geschwindigkeitsbestimmenden Schritt der Arzneistoffliberation durch folgende, teilweise simultan ablaufende Prozesse erheblich eingeschränkt:
- Eindringen von Wasser an der Oberfläche
- Ausbildung einer Gelschicht
- Auflösen des Wirkstoffes
- Diffusion des Wirkstoffes durch die Gelschicht
- Erosion der gelförmigen Matrix
- gleichzeitige Freisetzung des Wirkstoffes und Auflösung des Polymers

Die erfindungsgemäße Sol-gesteuerte Thermokolloid-Matrixtablette führt im Gegensatz dazu erstmalig zu einer Tablette, die die oben genannten Phänomene auf ein Minimum reduziert. So hängt die Arzneistoffliberation im zeitliche Verlauf nur noch von der an der Oberfläche fast nicht sichtbaren Sol-Schicht ab, deren Auflösung der geschwindigkeitsbestimmende Schritt ist. Diese Schicht ist zudem interessanterweise in der Lage, ein tieferes Eindringen von Wasser und damit ein Aufquellen der Matrix wirksam zu verhindern. Die in wäßrigem Milieu nicht aufquellende und stabile Tablette ist demzufolge auch in-vivo gegen mechanische Einflüsse bzw. Abrieb unempfindlich.

Grundsätzlich eignen sich alle handelsüblichen Gelatinesorten, die oberhalb 37°C kein Gelbildungsvermögen mehr besitzen und in einer Korngröße unterhalb 200 µm, vorzugsweise unterhalb 50 µm verpreßt werden. Das Gewichtsverhältnis von Arzneistoff zu Gelatine sollte nicht über 1:1 liegen, wobei dieses Verhältnis durch Spezialgelatine, die besonders peptidarm und hochviskos ist, auf 1:0,5 und mehr erhöht werden kann.

In einer weitergehenden Ausbildungsfom des erfindungsgemäßen Grundgedankens wird der Arzneistoff, berechnet auf die Tablettendosierung, in gelöster, suspendierter oder emulgierter Form in ein wäßriges oder mit organischen Lösungsmitteln versetztes Gelatinesol homogen eingebracht. Gelatinelösungen sind im Gegensatz zu den meisten natürlichen und synthetischen Hydrokolloiden auch bei höherer Konzentration im Vergleich eher niedrigviskos. Vorteilhafterweise kann daher das Solvens der obigen Gelatine/Arzneistoff-Mischung durch Sprühtrocknung problemlos und schonend entfernt werden.

Technologisch kann erfindungsgemäß hergestelltes Arzneistoff/Gelatinepulver mit oder ohne vorherige Granulation zu Tabletten oder Pellets verpreßt werden, die sich durch hohe Bruchfestigkeit und geringe Friabilität auszeichnen. Bei Anwendung der Sprühtrocknungstechnik mit anschließender Direkttablettierung liegt der optimale Korngrößenbereich zur Erzielung eines leicht rieselfähigen und gut verpreßbaren Pulvers unterhalb 200 µm, bevorzugt unterhalb 50 µm.

Zu der erfindungsgemäßen Zusammensetzung auf Gelatinebasis können bei Bedarf weitere Hilfsstoffe, Polymere synthetischer oder natürlicher Herkunft bzw. viskositätserhöhende Stoffe zugesetzt werden, sofern die Sol-gesteuerte Freigabe nicht eingeschränkt wird.

Die erfindungsgemäßen Tabletten oder Pellets können z.B. mit magensaftresistenten Filmbildnern und anderen pharmazeutisch bekannten Überzügen versehen werden.

Darüberhinaus können auch Manteltabletten zum Schutz und zur Steuerung der Freigabe von enzymatisch empfindlichen Arzneistoffen erfindungsgemäß hergestellt werden.

Die vorgeschlagene Verfahrensweise kann die Anwendungsbreite der vorgestellten Retardform erheblich erweitern. Weiterhin besitzt das Biopolymer Gelatine im Gegensatz zu anderen pharmazeutisch einsetzbaren Makromolekülen weitere interessante Eigenschaften, die erfindungsgemäß zusätzlich ausgenutzt werden:
- molgewichtsfraktionsabhängiges Verhalten
- amphiphile Eigenschaft und gutes Tensidbildungsvermögen
- pH-variierbare Ladungszustände
- steuerbare und temperaturabhängige Sol-Gel-Bildung

So lassen sich z.B. auch ohne weiteren Zusatz von Emulgatoren mit einem einzigen Hilfsstoff Emulsionen in sprühgetrockneter Form herstellen, die sich zur Direkttablettierung eignen. Damit können auch flüssige lipophile Arzneistoffe auf überraschend einfache Weise zu einer Retardarzneiform verarbeitet werden.

Als erfindungsgemäß einsetzbare Arzneistoffe eignen sich alle Arzneistoffe, die keine Inkompatibilität mit Gelatine aufweisen. Somit können flüssige und pastöse oder ölige wie auch leicht- und schwerlösliche Feststoffe eingesetzt werden.

Weiterhin können durch das Verfahren mangelhafter Geschmack wirksam verhindert und leicht flüchtige Stoffe vor Veränderung bewahrt werden.

Neben dem durch Sprüheinbettung zu erzielenden Schutz des Arzneistoffes vor Oxidation und Hydrolyse und damit eine Steigerung der Haltbarkeit ergeben sich auch technologische Vorteile. Die Homogenität und die sehr guten Direkt-Tablettiereigenschaften des erhaltenen Pulvers führen zu einer Vereinfachung des Herstellungsprozesses und verbessern die Chargenkonformität.

Erfindungsgemäß können folgende Arzneistoffe geeignet sein:

Aus der Gruppe der Analgetika/Antirheumatika, z.B.:
- Indometacin, Acemetacin
- Ibuprofen, Flurbiprofen, Ketoprofen
- Diclofenac
- ASS

Aus der Gruppe der Alkaloide, z.B.:
- Codein
- Dihydrocodein

Aus der Gruppe der Xanthin-Derivate, z.B.:
- Theophyllin, Diprophyllin

Aus der Gruppe der Beta-Blocker, z.B.:
- Pindolol
- Propranolol
- Metoprolol
- Oxprenolol

Aus der Gruppe der Antihypertonika, z.B.:
- Etilefrin, Renin-Antagonisten

Aus der Gruppe der Ca-Antagonisten, z.B.:
- Nifedipin und Strukturverwandte
- Diltiazem
- Verapamil

Aus der Gruppe der Antiarrhytmika, z.B.:
- Procainamid
- Prajmalin
- Disopyramid

Aus der Gruppe der Antitussiva, z.B.:
- Oxeladincitrat
- Dextromethorphan

Aus der Gruppe der durchblutungsfördenden Mittel, z.B.:
- Vincamin

Aus der Gruppe der Mucolytica, Broncholytika, Antiasthmatika z.B.:
- Ambroxol, Salbutamol

Aus der Gruppe der Vasodilatatoren, z.B.:
- Dihydroergotoxin

Aus der Gruppe der Koronartherapeutika, z.B.:
- Molsidomin, Salpetersäureester (Isosorbidmono- und -dinitrat)

Aus der Gruppe der Psychopharmaka, z.B.:
- Oxazepam

Aus der Gruppe der Antihistaminika, z.B.:
- Carbinoxamin

Aus der Gruppe der Vitamine, z.B.:
fett- und wasserlösliche Vitamine

Aus der Gruppe der Diuretica, z.B.:
- Furosemid

Aus der Gruppe der lipidsenkenden Arzneistoffe, z.B.:
- Bezafibrat, Fenofibrat, Xantinolnicotinat

Aus der Gruppe der Arzneistoffe zur Behandlung der erworbenen Immunschwäche AIDS, z.B.
- Renin-Antagonisten

Aus der Gruppe der Antibiotika/Chemotherapeutika, z.B.:
- Nitrofurantoin

Aus der Gruppe der Peptidarzneistoffe, z.B.:
- Insulin, Interferone, Renin-Antagonisten.
Wie schon in den eingangs erwähnten Patentanmeldungen ausführlich dargelegt, lassen sich mit dem erfindungsgemäßen Vorgehen vorteilhaft Resorptionsunterschiede ausgleichen und die Verträglichkeit steigern.

Besonders geeignet sind weiterhin höherdosierte Arzneistoffe, wie z.B. Schmerzmittel aus der Gruppe der nichtsteroidalen Antirheumatika (NSAR) und deren Enantiomere.

Weiterhin hat es sich interessanterweise gezeigt, daß in direkter Abhängigkeit der ausgewählten Gelatinesorte der Retardierungszeitraum in weiten Grenzen zu variieren ist. Kaltwasserlösliche Gelatinen, die ein Molekulargewicht unterhalb von 10⁴ D besitzen, sind zur Retardierung eher ungeeignet. Eine 1-2 stündige Retardierung kann durch niedrigbloomige bzw. niedrigviskose Gelatine eingestellt werden. Mittel- bis hochbloomige bzw. mittel- bis hochviskose Sorten verlängern linear entsprechend ihrer Molekulargewichtsverteilung den Zeitraum der vollständigen Arzneistoffliberation auf bis zu 16 Stunden und mehr.

Die Entwicklung und das Scaling-up einer Retardform, die einen für den entsprechenden Arzneistoff gewünschten Freigabezeitraum aufweist, kann durch einen weiteren erfindungsgemäßen Vorschlag standardisiert und reproduzierbar gemacht werden. Wählt man z.B. als geeignete Verfahrensweise eine Auflösung des Arzneistoffs in wässriger Gelatinelösung mit anschließender Sprüheinbettung, so kann ab einem kritischen Arzneistoff-Gelatineverhältnis diese Beladung zu einer Veränderung der chemisch-physikalischen Eigenschaften der eingesetzten Gelatine führen. So kann durch den "Aufsalzungseffekt" bei hoher Dosierung des Wirkstoffs eine an sich geeignete Gelatinesorte mit einem Retardierungsvermögen von beispielsweise 10 h eine entsprechend kürzere Freisetzungsdauer aufweisen.

Um diese Phänomene in der Vorausformulierung quantitativ meßbar zu machen, kann man erfindungsgemäß wie folgt vorgehen:

Durch Differentialthermoanalyse und Messung der Molekulargewichtsverteilung (HPLC) kann vorab eine Aussage getroffen werden, welche Gelatinesorte für den entsprechenden Anwendungsfall geeignet ist. Von der ausgewählten Gelatinesorte wird die Bloomzahl (Gelatinemonographie, Bloomtest nach DAB 9) bestimmt. Unter gleichen Testbedingungen und Konzentrationsverhältnissen erfolgt eine zweite Messung, bei dem der Arzneistoff in der vorausberechneten Konzentration in Gelatine gelöst vorliegt. Hierbei ergibt sich eine Differenz des Ausgangs-Bloomwertes. Die gemessene Erniedrigung des Bloomwertes zwischen erster und zweiter Messung dient als direktes Maß für die Wechselwirkung des gelösten Arzneistoffs mit Gelatine.

Hat die Ausgangsgelatine beispielsweise einen Bloomwert von 250 und führt die zweite Messung mit arzneistoffbeladener Gelatine zu einem Bloomwert von z.B. 220, so kann man durch Verwendung einer 270 Bloomgramm-Gelatine die ursprünglich gewünschte Freigabecharakteristik entsprechend Tabelle (Auflösungszeiten) aus Beispiel 1 erneut einstellen.

Ein analoges Verfahren läßt sich durch Vergleichsmessung der Viskosität darstellen, die insbesondere eine Aussage über die viskositätsbestimmende Gelatinemolfraktion oberhalb 10⁶ D zuläßt.

Die erfindungsgemäße Sol-gesteuerte Thermokolloidmatrix kann weiterhin für schwerlösliche Arzneistoffe mit problematischer Bioverfügbarkeit neue Lösungen anbieten, die sich aus der Matrixeinbettung in Nanosoltechnik der eingangs erwähnten Patentanmeldungen, insbesondere "Pharmazeutisch applizierbares Nanosol und Verfahren zu seiner Herstellung", "Ein 2-Aryl-propionsäurederivat in Nanosolform enthaltendes Arzneimittel und seine Herstellung", "Ein Dihydropyridinderivat in Nanosolform enthaltendes Arzneimittel und seine Herstellung" und "Perorale Applikationsform für Peptidarzneistoffe" ergeben. Der in Nanosolform vorliegende und stabilisierte schwerlösliche Arzneistoff wird nach der Trocknung, vorzugsweise Sprühtrocknung, zur entsprechenden Tablette verpreßt. Neben dem zu beachtenden Ladungszustand des Gelatine/Arzneistoff-Nanosols werden im Sinne der vorliegenden Erfindung Gelatinesorten ausgewählt, die entsprechend Vortest das gewünschte Retardierungspotential aufweisen. Der Vorteil des Verfahrens liegt neben den schon beschriebenen Eigenschaften der erfindungsgemäßen Matrix auch in dem Schutz des Nanosol-Arzneistoffes vor Abbau und vorzeitiger Instabilität durch in die Tablette eindringende gastrointestinale Flüssigkeit, wie sie z.B. bei quellenden Hydrogelmatrices auftreten können.

Insbesondere können somit schwerlösliche Arzneistoffe mit ausgeprägten Säure-Base-Eigenschaften zuverlässig unter Resorptionsverbesserung retardiert werden.

Ein weiterer Schutz vor vorzeitigem Abbau eines empfindlichen Arzneistoffes kann durch Mikroverkapselung unter Verwendung von zwei unterschiedlich geladenen Gelatinesorten erreicht werden. Hierbei schlägt die Erfindung folgendes Verfahren vor:

Der Arzneistoff wird zunächst durch Solvent Evaporation einer Mischung aus Gelatine Typ A und Typ B koazerviert. Die weitere Verarbeitung erfolgt erfindungsgemäß durch direkte Sprühtrocknung der erhaltenen Mischung ohne vorherige Abtrennung der Mikrokapseln. Das erhaltene Pulver kann bedingt durch den nicht abgetrennten und nicht koazervierten Gelatineanteil zu einer Sol-gesteuerten Thermokolloidmatrix in Tablettenform verarbeitet werden. Für diesen Spezialfall der Erfindung eignen sich bevorzugt Gelatinesorten, die einen Teil der Molfraktion oberhalb 300 kD von mindestens 20% aufweisen. Solche Gelatinesorten, insbesondere von sauer aufgearbeiteter Gelatine (Typ A), lassen sich nur durch spezielle Herstellungsverfahren gewinnen und zeichnen sich durch einen niedrigen Peptidanteil aus. Eine besonders homogene Verteilung der erhaltenen Mikrokapseln lassen sich durch Verwendung von Gelatine Typ A und B mit gleicher oder ähnlicher Molekulargewichtsverteilung erhalten.

Eine so hergestellte Arzneiform gibt den Wirkstoff in überwiegend mikroverkapselter Form geschützt und kontinuierlich ab und kann damit auch für Arzneistoffe, die einem enzymatischen Abbau im GIT unterliegen, wie z.B. Peptidarzneistoffe, vorteilhaft peroral angewendet werden. Die extrem hohe Viskosität der eingesetzten Gelatinesorten erleichtert zusätzlich die gewünschte Anreicherung an der Mucosa- bzw. Epithelschicht während der gastrointestinalen Passage.

Es ist bekannt, daß man eine sogenannte gepulste Freigabe in Tablettenform durch abwechselnd wirkstoffhaltige und wirkstofffreie Schichten z.B. mit Hydrokolloidmatrices erhalten kann.

Die Variationsbreite der Erfindung soll durch eine entsprechende Arzneiform demonstriert werden. Hierbei wirkt sich die bereits erwähnte im wäßrigen Milieu nicht quellende und stabile Tablettenoberfläche, die zu keiner unkontrollierbaren Erosion führt, vorteilhaft auf die zeitabhängige Dosierungsform aus. So können die wirkstoffhaltigen und wirkstofffreien Schichten z.B. wie folgt zusammengesetzt sein:

Wirkstoffhaltige Schichten sind aus erfindungsgemäß linear freisetzenden, Sol-gesteuerten Matrices aufgebaut, die durch Auswahl einer geeigneten Gelatinesorte den Wirkstoff z.B. in einer Stunde freigibt. Schichten ohne Wirkstoff können, sofern die gepulste Freigabe in kürzeren Abständen gewünscht ist, auf der Basis einer niedrig molekular zusammengesetzten Gelatine aufgebaut sein.

Auf diese Weise lassen sich stabile, nicht quellbare Tabletten mit drei- bis vierfach gepulster Freigabe herstellen. Sofern eine mehrfach gepulste Retardierung über einen längeren Zeitraum erwünscht ist, kann die damit zwangsläufig verbundene Verringerung der Schichtdicke (bedingt durch die maximal vertretbare Tablettengröße) der wirkstoffhaltigen und wirkstofffreien Zonen durch Auswahl von Gelatinesorten mit höherer Molekulargewichtszusammensetzung bzw. Viskosität kompensiert bzw. zeitabhängig gesteuert werden. Im Extremfall lassen sich sehr dünne, zeitverzögert und konstant auflösende wirkstofffreie Schichten zur gepulsten Steuerung ausbilden, die aus speziellen Gelatinesorten mit Sol-Bildungstemperaturen knapp unterhalb 37°C bestehen.

Auf diese Weise läßt sich trotz Mehrfachpulsierung eine stabile Tablette mit niedriger Steghöhe herstellen.

In analoger Weise kann eine solche Tablette auch mit zwei verschiedenen Arzneistoffen ohne wirkstofffreie Schichten aufgebaut sein. So kann man z.B. in der Rheumatherapie eine Kombination aus S- und R-Flurbiprofen oder anderen nichtsteroidalen Antirheumatika, die ein hohes Wirkstoffpotential besitzen, abwechselnd mit der schmerzstillenden (schnelle Anflutung) und der entzündungshemmenden (verlängerte Anflutung) Komponente aufbauen.

Um eine hohe Chargenkonformität bei dieser aufwendigen, jedoch aktuellen Arzneiform sicherzustellen und einen kontrollierten Aufbau der Schichten zu gewährleisten, sollte der Wirkstoff vorzugsweise in sprühgetrockneter Form eingearbeitet sein.

In Abhängigkeit von der Herstellungsweise von Gelatine (Ausmaß des Abbaus des nativen Kollagens und saures bzw. alkalisches Aufschlußverfahren) weist Gelatine vom Typ A oder Typ B ein charakteristisches Molekulargewichtsspektrum bzw. Molekulargewichtsverteilung auf. In Tabelle 1 sind die Molekulargewichtsverteilungen von verschiedenen Gelatinetypen bzw. von Kollagenhydrolysaten angegeben, sowie der prozentuale Anteil (Häufigkeit) einzelner Molekulargewichtsbereiche.

**Tabelle 1**

| Molekulargewichtsverteilung von verschiedenen bekannten Gelatinetypen bzw. von bekannten Kollagenhydrolysaten | | | | | | | |
|---|---|---|---|---|---|---|---|
| Molekulargewichtsverteilung (kD) | Natives Kollagen % | Gelatine Typ B % | Gelatine Typ A % | Kollagenhydrolysat Gelita ® Collagel A | Kollagenhydrolysat Gelita ® Collagel B | Kollagenhydrolysat Gelita ® Sol C | Elastinhydrolysat Gelita ® Gelastin |
| >360 | 100 | 18,0 | 18.0 | 0 | 0 | 0 | 0 |
| 285 | 0 | 7,0 | 9,0 | 0 | 0 | 0 | 0 |
| 145-237 | 0 | 20,0 | 34,0 | 1,0 | 1,5 | 0 | 0 |
| 95 | 0 | 26,0 | 11,0 | 0 | 0 | 0 | 0 |
| 95-50 | 0 | 16,3 | 13,4 | 2,6 | 4,0 | 1,1 | 0 |
| 50-20 | 0 | 7,4 | 9,1 | 18,0 | 14,5 | 0,3 | 0 |
| 20-10 | 0 | 3,9 | 3,8 | 43,0 | 31,5 | 3,7 | 0,2 |
| 10-5 | 0 | 3,0 | 3,0 | 15,4 | 20,0 | 12,2 | 5,2 |
| 5-2 | 0 | 0 | 0 | 6,0 | 14,0 | 26,0 | 93,9 |
| 2-1 | 0 | 0 | 0 | 7,0 | 8,0 | 23,0 | 0 |
| < 1 | 0 | 0 | 0 | 6,3 | 7,0 | 34,0 | 0 |
| MG | 360 | 165 | 185 | 12-18 | 12-18 | 3 | 2-3 |

Man erkennt in den einzelnen Spalten deutlich das Überwiegen eines einzelnen Bereiches im Vergleich zu den übrigen Molekulargewichtsbereichen derselben Gelatine. Dieser Bereich stellt also das Maximum der Molekulargewichtsverteilung dar (es liegt z.B. bei der in der Abbildung aufgeführten Gelatine Typ B bei 95 kD). Der Begriff des "Maximums der Molekulargewichtsverteilung" ist jedoch streng zu trennen von dem Begriff des "durchschnittlichen mittleren Molekulargewichts". Dieser Mittelwert liegt bei der erwähnten Gelatine vom Typ B bei 165 kD.

Bei der Formulierung von Arzneimitteln macht der Pharmazeut einen grundsätzlichen Unterschied zwischen:
1. galenischer Zubereitung, d.h. einer Freisetzung des Arzneistoffes, z.B. aus einer Tablette in zeitlich schneller (Akutform) oder verlangsamter (Retardform) Weise;
   und
2. dem arzneistoffspezifischen Resorptionsort, wie z.B. Magen oder bestimmte Darmabschnitte.

Die erfindungsgemäßen Nanosole sind in der Lage, unabhängig von der galenischen Zubereitung, aufgrund ihrer speziellen Zusammensetzung, im gesamten gastrointestinalen Bereich resorbiert zu werden. Sie können daher vorteilhaft zu Akut- bzw. Retardarzneiformen weiterverarbeitet werden.

Arzneistoffe mit problematischer bzw. eingeschränkter Bioverfügbarkeit lassen sich durch die im beschreibenden Teil der Erfindung angegebenen Ausführungsformen wie z.B. Nanosoltechnik vorteilhaft zur gepulsten Freisetzung anwenden.

Folgende Beispiele sollen die Erfindung näher erläutern:

### Beispiel 1:

Gelatinesorten mit Bloomwerten von 50, 150, 200, 250, 300 und 350 werden bei 50°C in Konzentrationen von 5-10% wie üblich aufkonzentriert bzw. sprühgetrocknet. Das Pulver wird granuliert und mit einer Restfeuchte von 12% zu Tabletten mit einem Gewicht von 400 mg auf einer Exzenterpresse unter Zusatz von FST-Komplex direkt verpreßt. Nach USP XXI (paddle-Apparatur) werden die durchschnittlichen Auflösungszeiten von jeweils 6 Tabletten in 900 ml Wasser, bei 100 UpM und 37°C bestimmt. Die gefundenen Auflösungszeiten sind aus der Tabelle zu entnehmen. Ähnliche Auflösungszeiten erhält man mit Tabletten aus feinstgemahlenem Gelatinepulver (Korngröße unterhalb 200 µm) der o.g. Qualitäten.

Tabelle Auflösungszeiten:

| Bloomwert (g) | Auflösungszeiten (min) |
|---|---|
| 50 | 55 |
| 150 | 120 |
| 200 | 178 |
| 250 | 265 |
| 300 | 413 |
| 350 | 627 |

### Beispiel 2:

500 g Gelatine mit einem Bloomwert von 300 werden in 10 l Wasser bei 50°C gelöst. Anschließend werden in dem Sol 10 g Methylenblau gelöst, die Lösung konzentriert, sprühgetrocknet und analog Beispiel 1 zu Tabletten von 300 mg verpreßt. In einer Freisetzungsapparatur (paddle) nach USP XXI wird unter folgenden Bedingungen die Freisetzung des Farbstoffes von jeweils 6 der 300 mg-Tabletten bestimmt: 900 ml Wasser, 50 UpM, 37°C. Es wird zu bestimmten Zeiten jeweils 1 ml Probelösung entnommen und VIS-spektroskopisch der Gehalt an freigesetztem Methylenblau bei 663 nm bestimmt. Die erhaltenen Werte sind aus der Tabelle zu entnehmen.

Tabelle Freisetzung:

| Zeit [h] | Freisetzung in % |
|---|---|
| 0,25 | 8 |
| 0,5 | 13 |
| 1 | 22 |
| 1,5 | 33 |
| 2 | 42 |
| 3 | 58 |
| 4 | 67 |
| 5 | 83 |
| 6 | 98 |

Unter gleichen Bedingungen hergestellte und gemessene Tabletten, mit Gelatine der Bloomkennzahl 330, ergeben eine 100%ige Freisetzung nach 485 min.

Die beschriebenen Tablettenchargen zeigen gegenüber Beispiel 1 eine kürzere Auflösezeit, die in diesem Fall vom niedrigeren Tablettengewicht abhängig ist.

### Beispiel 3:

Da es sich bei Verapamil-HCl um ein wasserlösliches Salz handelt, wird der Vortest zur Bloomdepression folgendermaßen durchgeführt:

Eine Gelatine nach Beispiel 1 mit einer Auflösungszeit von 413 Minuten ergibt nach dem Bloomtest nach DAB 9 (7,5 g Gelatine in 105 ml Wasser) einen Wert von 300 Bloom. In einem zweiten Versuch werden zu einer gleich konzentrierten Gelatinelösung 2,37 g Verapamil HCl gegeben, darin gelöst und der Test erneut nach Vorschrift durchgeführt. Es werden 280 Bloom ermittelt. Um die Erniedrigung der Gelfestigkeit zu kompensieren und die gewünschte Auflösungszeit zu erhalten, wird eine Gelatine mit 315 Bloom eingesetzt.

0,12 kg Verapamil-HCl werden in eine aus 0,38 kg oben spezifizierter Gelatine und 8 l Wasser bei 50°C hergestellten Gelatinelösung eingerührt. Die erhaltene klare Lösung wird sprühgetrocknet und mit einer mittleren Korngröße von 50-60µm unter Zusatz von FST-Komplex auf einer Exzenterpresse zu Tabletten mit einer Dosierung von 120 mg und 500 mg Gesamtgewicht gepreßt.

Der Dissolution-Test (paddle) bei 37°C, 900ml Wasser und 50 UpM mit einem Wechsel des Prüfmediums von künstlichen Magensaft zu künstlichem Darmsaft nach 2 Stunden ergibt durchschnittlich folgende pH-unabhängige Freisetzungsdaten:

| Zeit (h) | Freisetzung in % |
|---|---|
| 1 | 24 |
| 2 | 34 |
| 3 | 56 |
| 4 | 65 |
| 5 | 82 |
| 6 | 95 |
| 7 | 100 |

### Beispiel 4:

Propranolol-HCl-Tabletten mit 500 mg Tablettengewicht und einer Dosierung von 80 mg sollen hergestellt werden. Als Gelatinequalität werden 290 Bloom eingesetzt, die sich aus dem gewünschten Retardierungszeitraum (s. Beispiel 1) und analog Vortest (Bloomkompensation, s. Beispiel 3) ergibt.

80 g Propranolol-HCl werden in 0,42 kg Gelatine obiger Qualität und 5 l Wasser bei 50°C gelöst. Anschließend wird wie unter Beispiel 3 beschrieben, die Tablettencharge hergestellt.

Der Dissolutiontest nach USP XXI (Drehkörbchen) in 1000 ml verdünnter HCl, 37°C und 100 UpM ergibt folgende durchschnittliche Werte:

| Zeit (h) | Freisetzung in % |
|---|---|
| 1 | 16 |
| 2 | 39 |
| 3 | 52 |
| 4 | 73 |
| 5 | 91 |
| 6 | 100 |

## Patentansprüche

1. Perorales Depotarzneimittel, gekennzeichnet durch ein in wässrigem Medium oberhalb 37°C pro Zeiteinheit annähernd konstant Arzneistoff freisetzendes Komprimat, das im wesentlichen aus einer Matrix aus Gelatine oder fraktionierter Gelatine bzw. deren Mischungen und darin verteiltem Arzneistoff besteht.

2. Perorales Depotarzneimittel nach Anspruch 1, dadurch gekennzeichnet, daß die Gelatine, fraktionierte Gelatine und/oder deren Mischung ein Maximum der Molekulargewichtsverteilung haben, das im Bereich von 10⁴ bis 10⁷ D liegt.

3. Perorales Depotarzneimittel nach Anspruch 1 und/oder 2, dadurch gekennzeichnet, daß die Gelatine Kennzahlen von 50 bis 400 Bloom aufweist.

4. Perorales Depotarzneimittel nach einem der Ansprüche 1 bis 3, dadurch gekennzeichnet, daß die Matrix aus einem Komprimat besteht, das neben Gelatine synthetische und/oder natürliche Polymere und weitere Hilfsstoffe enthält.

5. Perorales Depotarzneimittel nach einem der Ansprüche 1 bis 4, dadurch gekennzeichnet, daß im Arzneimittel eine oder mehrere arzneistoffhaltige mit einer oder mehreren arzneistoffreien Schicht(en) oder Schichten mit unterschiedlichen Arzneistoffen abwechseln.

6. Perorales Depotarzneimittel nach einem der Ansprüche 1 bis 5, dadurch gekennzeichnet, daß es mit einem Überzug aus einem synthetischem oder natürlichem Polymeren versehen ist.

7. Perorales Depotarzneimittel nach einem der Ansprüche 1 bis 6, dadurch gekennzeichnet, daß es mit einer Akutform kombiniert vorliegt.

8. Verfahren zur Herstellung eines peroralen Depotarzneimittels nach Anspruch 1, dadurch gekennzeichnet, daß man mit einer Gelatine und/oder fraktionierter Gelatine, die sich im wässrigen Medium oberhalb 37°C langsam auflöst, eine pulverförmige Gelatine-Arzneistoff-Mischung herstellt und die Mischung komprimiert.

9. Verfahren nach Anspruch 8, dadurch gekennzeichnet, daß man die Gelatine-Arzneistoff-Mischung im wesentlichen aus Gelatine und/oder fraktionierter Gelatine neben synthetischen und/oder natürlichen Polymeren und weiteren Hilfsstoffen sowie dem Arzneistoff herstellt.

10. Verfahren nach Anspruch 8 und/oder 9, dadurch gekennzeichnet, daß man die Mischung herstellt, indem man pulverförmige Gelatine und pulverförmigen Arzneistoff homogen vermischt.

11. Verfahren zur Herstellung eines peroralen Depotarzneimittels nach Anspruch 1, dadurch gekennzeichnet, daß man die Gelatine-Arzneistoff-Mischung direkt zu einer Tablette komprimiert.

12. Verfahren nach Anspruch 8, dadurch gekennzeichnet, daß man die Gelatine-Arzneistoff-Mischung herstellt, indem man die in Solform vorliegende Gelatine mit dem Arzneistoff versetzt und anschließend sprühtrocknet.

13. Verwendung eines in wässrigem Medium oberhalb 37°C pro Zeiteinheit annähernd konstant Arzneistoff freisetzenden Komprimates aus einer Matrix aus Gelatine und/oder fraktionierter Gelatine und darin verteiltem Arzneistoff zur Herstellung einer peroralen Depotarzneiform.

## Claims

1. Oral depot medicament, characterised by a compressed tablet which releases pharmaceutical in an approximately constant manner per time unit in aqueous medium above 37°C, which essentially consists of a matrix of gelatin or fractionated gelatin or mixtures thereof and pharmaceutical dispersed therein.

2. Oral depot medicament according to Claim 1, characterised in that the gelatin, fractionated gelatin and/or mixture thereof have a maximum in the molecular weight distribution which is in the range from 10⁴ to 10⁷ D.

3. Oral depot medicament according to Claim 1 and/or 2, characterised in that the gelatin has characteristic numbers of 50 to 400 bloom.

4. Oral depot medicament according to one of Claims 1 to 3, characterised in that the matrix consists of a compressed tablet which in addition to gelatin contains synthetic and/or natural polymers and other auxiliaries.

5. Oral depot medicament according to one of Claims 1 to 4, characterised in that, in the medicament, one or more pharmaceutical-containing layer(s) alternate with one or more pharmaceutical-free layer(s) or layers of different pharmaceuticals.

6. Oral depot medicament according to one of Claims 1 to 5, characterised in that it is provided with a coating of a synthetic or natural polymer.

7. Oral depot medicament according to one of Claims 1 to 6, characterised in that it is present combined with an immediate-effect form.

8. Process for the production of an oral depot medicament according to Claim 1, a powdered gelatin/pharmaceutical mixture is prepared using a gelatin and/or fractionated gelatin which dissolves slowly in the aqueous medium above 37°C and the mixture is compressed.

9. Process according to Claim 8, characterised in that the gelatin/pharmaceutical substance mixture is essentially prepared from gelatin and/or fractionated gelatin in addition to synthetic and/or natural polymers and other auxiliaries as well as the pharmaceutical.

10. Process according to Claims 8 and/or 9, characterised in that the mixture is prepared by homogeneously mixing powdered gelatin and powdered pharmaceutical.

11. Process for the production of an oral depot medicament according to Claim 1, characterised in that the gelatin/pharmaceutical mixture is compressed directly to give a tablet.

12. Process according to Claim 8, characterised in that the gelatin/pharmaceutical mixture is prepared by mixing the gelatin present in sol form with the pharmaceutical and then spray-drying.

13. Use of a compressed tablet which releases pharmaceutical in an approximately constant manner per time unit in aqueous medium above 37°C and is made from a matrix of gelatin and/or fractionated gelatin and pharmaceutical dispersed therein for the production of an oral depot pharmaceutical form.

## Revendications

1. Médicament peroral à action retardée, caractérisé par un comprimé libérant la substance médicamenteuse en quantité approximativement constante par unité de temps en milieu aqueux au-dessus de 37°C, qui est principalement constitué d'une matrice de gélatine ou de gélatine fractionnée ou de leurs mélanges et d'une substance médicamenteuse qui y est répartie.

2. Médicament peroral à action retardée suivant la revendication 1, caractérisé en ce que la gélatine, la gélatine fractionnée et/ou leur mélange ont un poids moléculaire dont le maximum de répartition se situe dans la plage de 10⁴ à 10⁷ D.

3. Médicament peroral à action retardée suivant la revendication 1 et/ou la revendication 2, caractérisé en ce que la gélatine présente des indices de 50 à 400 Bloom.

4. Médicament peroral à action retardée suivant l'une des revendications 1 à 3, caractérisé en ce que la matrice est constituée d'un comprimé qui contient, en plus de gélatine, des polymères synthétiques et/ou naturels et d'autres substances auxiliaires.

5. Médicament peroral à action retardée suivant l'une des revendications 1 à 4, caractérisé en ce qu'une ou plusieurs couches contenant la substance médicamenteuse alterne avec une ou plusieurs couches sans substance médicamenteuse ou des couches contenant des substances médicamenteuses différentes.

6. Médicament peroral à action retardée suivant l'une des revendications 1 à 5, caractérisé en ce qu'il est enrobé d'un revêtement formé d'un polymère synthétique ou naturel.

7. Médicament peroral à action retardée suivant l'une des revendications 1 à 6, caractérisé en ce qu'il est associé à une forme à action rapide.

8. Procédé de production d'un médicament peroral à action retardée suivant la revendication 1, caractérisé en ce qu'on prépare un mélange en poudre de gélatine et de substance médicamenteuse avec une gélatine et/ou une gélatine fractionnée, qui se dissolvent lentement en milieu aqueux au-dessus de 37°C et on comprime le mélange.

9. Procédé suivant la revendication 8, caractérisé en ce qu'on prépare le mélange de gélatine et de substance médicamenteuse principalement à partir de gélatine et/ou de gélatine fractionnée, à côté de polymères synthétiques et/ou naturels et d'autres substances auxiliaires ainsi que de la substance médicamenteuse.

10. Procédé suivant la revendication 8 et/ou la revendication 9, caractérisé en ce qu'on prépare le mélange en formant un mélange homogène de gélatine en poudre et de substance médicamenteuse en poudre.

11. Procédé de production d'un médicament peroral à action retardée suivant la revendication 1, caractérisé en ce qu'on presse directement en un comprimé le mélange de gélatine et de substance médicamenteuse.

12. Procédé suivant la revendication 8, caractérisé en ce qu'on prépare le mélange de gélatine et de substance médicamenteuse en ajoutant la substance médicamenteuse à la gélatine se présentant sous forme de sol et en séchant ensuite le mélange par pulvérisation.

13. Utilisation d'un comprimé libérant une quantité à peu près constante de substance médicamenteuse par unité de temps en milieu aqueux au-dessus de 37°C, formé d'une matrice de gélatine et/ou de gélatine fractionnée dans laquelle la substance médicamenteuse est répartie, pour la préparation d'une forme perorale de médicament à action retardée.
